Europäisches Patentamt

European Patent Office

Office européen des brevets

(11) Publication number: 0 317 847
A1

## (12) EUROPEAN PATENT APPLICATION

(21) Application number: 88118824.7

(22) Date of filing: 11.11.88

(51) Int. Cl.⁴: A61B 5/14 , G01N 33/48

Amended claims in accordance with Rule 86 (2) EPC.

(30) Priority: 17.11.87 US 121673

(43) Date of publication of application:
31.05.89 Bulletin 89/22

(84) Designated Contracting States:
CH DE FR GB IT LI

(71) Applicant: SPACELABS, INC.
4200- 150th Avenue N.E.
Redmond Washington 98073-9713(US)

(72) Inventor: Pelikan, Glenn W.
2836 N.E. 54th
Portland Oregon 97213(US)
Inventor: Lombardi, Carl A.
13305 N. E. 51st Place
Bellevue Washington 98005(US)

(74) Representative: Patentanwälte Grünecker,
Kinkeldey, Stockmair & Partner
Maximilianstrasse 58
D-8000 München 22(DE)

(54) Apparatus and method for blood chemistry analysis.

(57) A device (100) comprises a hypodermic syringe (102) having a needle (106) attached to a piston (120)/ cylinder (108) arrangement and a sensor (140, 142, 144) disposed to come in contact with blood drawn into the cylinder for sensing at least one blood component. In one embodiment, a sensing surface (146, 148, 150) of the sensor forms a part of the interior surface of the cylinder. In another embodiment, the device includes a smaller cylinder (312) having a coaxial interior channel (314) open at both ends of the smaller cylinder. One end of the cylinder is sealably attached to the end of the syringe cylinder (308) to which the needle (306) is attached. The sensor (340, 342, 344) is located within the wall of the smaller cylinder (312) and its sensing surface (346, 348, 350) is in communication with the coaxial channel (314). The sensor may protrude into the coaxial channel. The sensor may sense more than one component of the blood simultaneously and be comprised of a plurality of separate sensors. In other embodiments, the sensor is mounted in either an adapter (500) disposed between the needle (504) and syringe (502) or in an enlarged portion of the syringe. The hypodermic syringe is inserted into an artery of a patient and blood is drawn toward the syringe until the blood comes in contact with the sensor(s). After the data is collected the blood is ejected back into the blood stream. Needle sizes as small as twenty-two gauge may be used.

FIG.1

# APPARATUS AND METHOD FOR BLOOD CHEMISTRY ANALYSIS

## Technical Field

This invention relates to a method and apparatus for performing blood chemistry analysis, and more particularly to a bedside analysis in essentially realtime without causing significant blood loss to the patient.

## Background Art

Currently, when blood chemistry analysis is required, blood must be withdrawn from the patient using a hypodermic needle. The blood is then sent to the lab for analysis, and, after analysis, the blood is discarded. Typically, the blood is withdrawn from an accessible artery, such as the radial artery. In neonatals, this blood loss can be particularly traumatic.

Another disadvantage of the current procedure is the delay in obtaining results from the lab. There is, of course, an initial delay in getting the blood sample from the patient to the lab. Hospital labs are often very busy, and there can be a significant backlog resulting in a significant delay before the lab can analyze the blood sample. Finally, there is a delay in transmitting the test results to the physician. While these delays are often merely an inconvenience, there are times when rapid test results can be vital to the status of a patient.

One conventional approach to obtaining rapid blood chemistry test results utilizes blood chemistry sensors mounted in the tip of a catheter that is inserted into an artery. However, such catheters must be larger than 18 gauge in diameter in order to house the sensor. Catheters that are this large are often unusable in many applications.

## Disclosure of Invention

An object of the present invention is to provide for an improved method and apparatus for blood chemistry analysis.

Another object of the invention is to provide for the above- mentioned method and apparatus for use with neonates to minimize blood loss and trauma.

Another object of the invention is to provide a method and apparatus for allowing instantaneous blood chemistry analysis at the patient's bedside.

A still further object of the invention is to provide a method and apparatus for blood chemistry analysis which inherently prevents the blood sample from being contaminated by oxygen or other contaminants.

These and other objects of the invention are provided by a hypodermic syringe having a small diameter, hollow needle. Sensing means for sensing the chemical makeup of the blood is disposed to come in contact with the patient's blood drawn into the needle or the syringe. The sensing means is coupled by electrical leads to appropriate electronics for obtaining the information from the sensing means.

In one embodiment, the sensing means comprises a plurality of different separate sensors whose sensing surfaces make up a portion of the interior wall of the syringe. The sensors are individually coupled by a plurality of leads to a multilead connector which couples the leads to the electronics through a single multilead cable.

In another embodiment of the invention, the sensory means is mounted in a cylindrical adaptor that is connected between a conventional needle and syringe. With this embodiment, conventional syringes and needles can be used for blood chemistry analysis.

Similarly, in still another embodiment of the invention, the sensing means is mounted in an enlarged portion of a needle away from its distal end. The needle may then be mounted on conventional syringes.

The hypodermic syringe used with any of the embodiments is otherwise conventional having a glass plastic piston/cylinder arrangement for changing the volume of the interior chamber. The needle is first inserted in the artery of the patient. The piston is withdrawn drawing blood into the chamber in contact with the sensor surfaces. After the readings are taken under control of the electronic read out device, the piston is pressed inwardly in the chamber to eject the blood back into the patient and the needle is withdrawn. Because a very small needle, e.g. 22 gauge, is used, there is very little loss of blood and very little trauma to the patient. Further, since the analysis is made as the blood is withdrawn, it cannot be contaminated with oxygen or another gas, fluid or solid prior to the analysis.

## Brief Description of the Drawings

Figure 1 is a partial cross-sectional view of one embodiment of the invention in a first state coupled to control electronics.

Figure 2 is a partial cross sectional view of the embodiment of Figure 1 in a second state and coupled to control electronics.

Figure 3 is a cross section of an alternate embodiment of the invention.

Figure 4 is an isometric view of a sensing device used in the embodiment of Figure 3.

Figure 5 is a cross section of another embodiment of the invention which allows the inventive blood gas sensor to be used with conventional syringes.

Figure 6 is a cross-sectional view of still another embodiment of the invention.

Best Mode for Carrying Out the Invention

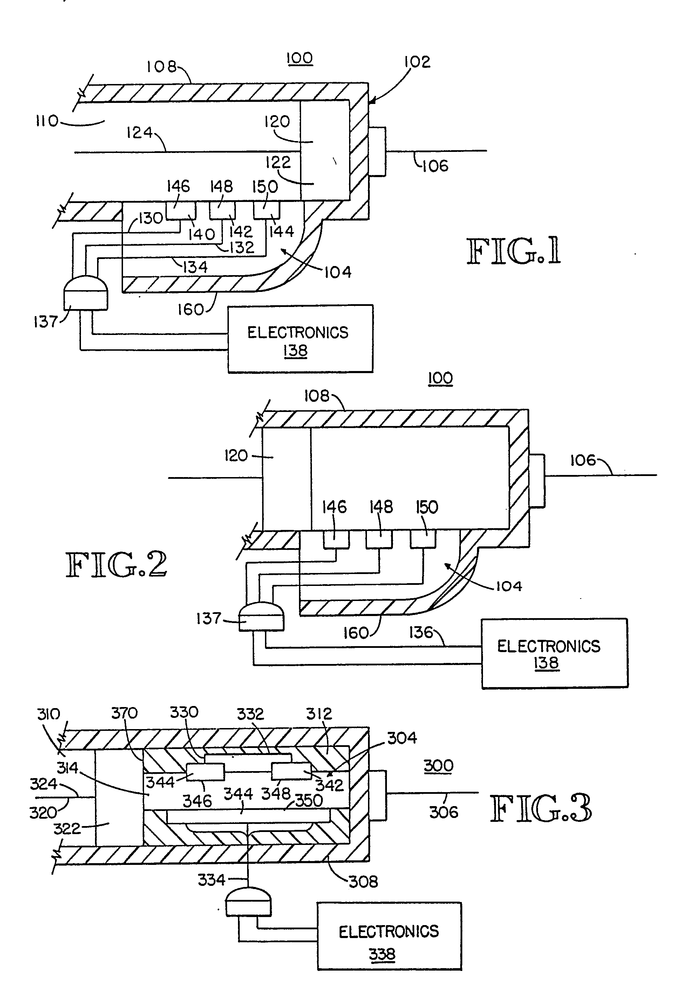

Referring now to Figures 1 and 2, a blood chemistry analysis device designated generally 100 comprises a hypodermic syringe 102 and a means 104 coupled to the syringe 102 for sensing components of the blood. The syringe further comprises a hollow needle 106 attached to a hollow cylinder 108 defining an interior chamber 110. The hollow interior of the needle 106 communicates with the chamber 110. The size of the needle 106 can be any size suitable for use with a hypodermic syringe. Although the cylinder 108 is shown as being circular in cross-section, it will be understood that the cylinder need not be circular, and other configurations may also be used.

The syringe further comprises a piston 120 having a disc or short cylinder 122 closely fitted in the hollow cylinder 108 and moved back and forth therein by a rod 124 connected thereto. The cylinder is typically transparent, made of glass or plastic.

The sensing means 104 is disposed to come in contact with the blood drawn into chamber 110 as the piston 120 is withdrawn as shown in Figure 2. The sensing means is coupled via leads 130, 132 and 134 and cable 136 to a conventional electronic readout device 138.

In one embodiment, the sensing means comprises a plurality of different separate sensors 140, 142 and 144 whose sensing surfaces 146, 148 and 150, respectively, make up a portion of the interior wall of cylinder 108 defining chamber 110. The sensors are contained within an enclosure 160 which is coupled to cylinder 108 and forms an enlarged compartment therein to house the sensors. The leads 130, 132 and 134 are coupled to cable 136 via connector 137 and the enclosure 160 is sealed where the leads 130, 132 and 134 pass through the enclosure wall. This protects the sensors from contamination.

Sensors suitable for use with the present invention are manufactured by Ionetics, Inc., having a principal place of business at 3020 Enterprise Street, Costa Mesa, California 92626. These sensors are ion selective membrane sensors capable of sensing a multiple number of blood constituents such as $O_2$, $CO_2$, pH, pottasium ions, calcium ions, sodium ions, as well as other ions and compounds including glucose, enzymes, etc. The sensors 140, 142 and 144 may be mounted in the cylinder 108 of the syringe 102 by any suitable means such as by molding the cylinder around the sensors 140, 142 and 144.

Figure 3 shows an alternate embodiment of the present invention device designated generally 300, comprising a hypodermic syringe 302 and means 304 for sensing components of the blood. The syringe 302 further comprises a hollow needle 306 attached to a hollow cylinder 308 defining an interior chamber 310. Like the embodiment of Figures 1 and 2, the size of the needle can be any size suitable for use with a hypodermic syringe.

The sensing means 304 comprises a smaller cylinder 312 with a central coaxial cylindrical chamber 314 which is in communication with the interior of needle 306 and the chamber 310. The outside diameter of the smaller cylinder is smaller than the inside diameter of the hypodermic needle cylinder 308 so the smaller cylinder 312 fits inside the cylinder 308. In the preferred embodiment, the smaller cylinder 312 forms a tight fit within the larger cylinder and comes in contact with the front wall 316 of the larger cylinder 308. The smaller cylinder may even be glued in place or otherwise attached so that the contact between the cylinders is sealed and blood withdrawn from the patient passes from the needle into the chamber 314.

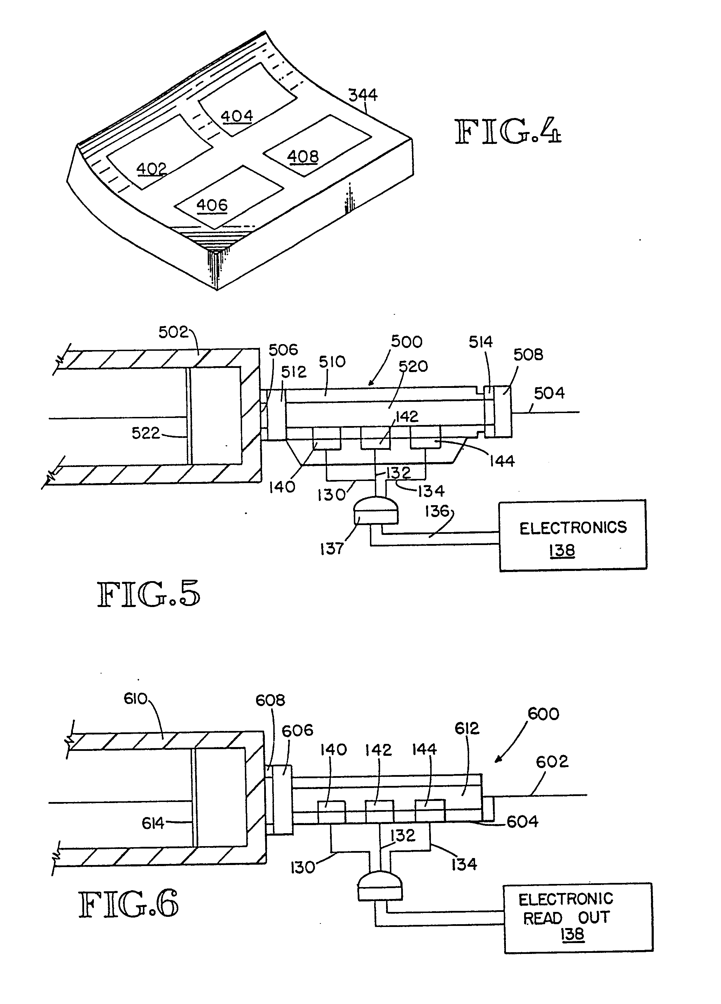

The wall of the smaller cylinder 312 is thick enough to accommodate the sensing means 304 which may be comprised of a plurality of individual sensing elements 340 and 342 like those described in Figures 1 and 2, or it may be a single sensing device 344 which is capable of sensing a plurality of blood components simultaneously from a plurality of different areas on the sensing surface. See, for example, sensing surfaces in numbers 402 through 408 in Figure 4. Sensors 340 and 342 are shown with leads 330 and 332 passing through the wall of cylinder 308 for coupling to a conventional electronics read out device 338 while the sensing device 344 has a plurality of leads 334 coupled thereto which pass through cylinder 308 to electronics 338. The syringe further comprises a piston 120 having a disc or short cylinder 322 closely fitted in the hollow cylinder 308 and moved back and forth therein by a rod 324 connected thereto.

The sensors 340, 342 and 344 are disposed to come in contact with the blood drawn into chamber 314 as the piston 320 is withdrawn. In Figures 1 and 2 as the piston is moved back and forth it must pass by the sensor surfaces 146, 148 and 150 and for that reason the surfaces must not protrude into the chamber 110. No such requirement exists for the embodiment of Figure 3, since the piston does not move within the chamber 314 but stops at the end 370 of the small cylinder. Hence, the sensing surface of the sensors may protrude into the chamber 314. The sensors 340 and 342 are shown with surfaces 346 and 348 protruding into the chamber 314. Even though sensing device 344 is shown with its surface 350 flush with the interior surface of cylinder 312, it too could protrude into the chamber 314. It should also be understood that the single sensing device 344 of Figure 3 could be used to replace the individual sensors of Figures 1 and 2.

In the embodiment illustrated in Figure 5, the blood chemistry sensors 140, 142 and 144 are mounted in a cylindrical adapter 500 connected between a conventional syringe 502 and a conventional needle 504. As well understood in the art, conventional needles 504 are typically mounted on conventional syringes 502 utilizing a LEURLOK® connector having interlocking portions on the syringe 506 and the needle 508. The adaptor 500 comprises an elongated cylinder 510 having a needle type LEURLOK® connector 512 at one end and a syringe-type LEURLOK® connector 514 at the other end. The connector 512 of the adapter 500 is thus secured to the connector 506 of the syringe 502 in the same manner as the needle 504 would be directly mounted on the syringe 502. Similarly, the connector 508 of the needle 504 is secured to the connector 514 of the adapter 500 in the same manner that it would be mounted on the connector 506 of the syringe 502.

The cylinder 510 forms an elongated cylindrical cavity 520 through which blood flows when a piston 522 in the syringe 502 moves to the left, as illustrated in Figure 5. The sensors 140, 142 and 144 then make contact with the blood in order to provide appropriate signals to the conventional electronic readout device 138. The primary advantages of the embodiment of Figure 5 are two-fold. First, the adapter 500 allows blood chemistry analysis using conventional syringes employing LEURLOK® connectors. Second, the relatively small diameter of the cavity 520 in the adapter 500 allows the blood chemistry analysis to occur without using a significant amount of blood.

In another embodiment, illustrated in Figure 6, the sensors 140, 142, 144 are mounted within a specially configured needle 600. The needle 600 has a relatively thin shaft 602 of conventional design at its distal end, an enlarged portion 604 in its midsection and a conventional LEURLOK® connector 606 at its proximal end. The LEURLOK® connector 606 is mounted on a conventional LEURLOK® connector 608 formed at the end of a conventional syringe 610. The enlarged portion 604 of the needle 600 forms an elongated cylindrical cavity 612 that communicates the sensing surface of the sensors 140, 142 and 144. In operation, when a piston 614 in the syringe 610 moved to the left, as illustrated in Figure 6, blood is drawn through the distal end of the needle 602 into the large portion 604 where it makes contact with the sensors 140, 142 and 144.

Because of the relatively small size of the cavity 612, the blood chemistry test can be made using an insignificant amount of blood. Furthermore, the needle 600 can be mounted on conventional syringes 610 and it can be configures to a variety of needle diameters, depending upon its use.

In use the needle of the syringe is inserted into an artery or vein of the patient such as the radial artery. The piston, which is in the forward most position when the needle is inserted, is withdrawn drawing blood into the cylinder portion of the syringe in the embodiments of Figures 1-3. Withdrawing the piston fills the cavity in the adapter in the embodiment of Figure 5 and the enlarged portion of the needle in the embodiment of Figure 6. In either case, withdrawing the piston brings the blood into contact with the sensors. The sensors convey data back to the electronic read out device where the data is analyzed. Once the date is collected, the blood may be ejected back into the patient's blood stream by re-inserting the piston.

As mentioned above, the electronic read out device is conventional and is not shown in detail here. One acceptable system for analyzing blood composition data is the electrolyte analyzers sold by Ionetics.

## Claims

1. A blood chemistry analysis device, comprising:
a hypodermic syringe having a hollow needle coupled to a hollow cylinder which forms a chamber whose volume is regulated by a piston movable within the hollow cylinder;
an enclosed cavity communicating with said syringe and needle so that blood drawn toward said syringe enters said cavity; and
a blood chemistry sensor mounted in said cavity to make contact with the blood entering said cavity, thereby allowing said blood to be chemically analyzed.

2. The blood chemistry analysis device of claim 1 wherein said cavity is formed by a portion of the hollow cylinder of said syringe.

3. The device of claim 2 wherein said sensor comprises a sensing surface which forms a part of the interior chamber of said syringe and is disposed to allow said piston to move back and forth across the sensing surface.

4. The device of claim 3 wherein said sensor comprises a plurality of separate sensors each with a sensing surface forming a part of said interior chamber.

5. The device of claim 1 wherein said sensor comprises a single sensing device sensing a plurality of blood components.

6. The device of claim 2 wherein said hypodermic syringe further includes a second cylinder having a coaxial channel running the entire length of said second cylinder an open at both of its ends, said second cylinder disposed to fit within said hollow cylinder of said syringe, said sensor located within the wall of said cylinder and exposed to the coaxial channel.

7. The device of claim 6 wherein said sensor protrudes into said coaxial channel.

8. The device of claim 6 wherein said sensor comprises a single sensing device sensing a plurality of blood components.

9. The device of claim 6 wherein a first end of said second cylinder is sealably attached to the end of said syringe cylinder so that said needle and said coaxial channel are in communication and said piston is movable from within the interior chamber of said syringe cylinder from a position engaging an end of said smaller cylinder opposite said first end to a remote position spaced apart from said opposite end.

10. The device of claim 1 wherein said cavity is formed in a hollow generally cylindrical adapter disposed between said syringe and needle with said sensor being mounted on said cylindrical adapter.

11. The device of claim 10 wherein said adapter has a syringe type connector at one end to which said needle is secured, and a needle type connector at its other end to which said syringe is secured.

12. The device of claim 10 wherein said sensor is mounted in the generally cylindrical wall of said adapter, said sensor having a sensing surface communicating with the cavity formed by said cylindrical adapter.

13. The device of claim 1 wherein said cavity is formed by an enlarged portion of said needle intermediate its distal and proximal ends, said sensor being mounted on the enlarged portion of said needle.

14. The device of claim 1 wherein said needle is substantially no larger than twenty-two gauge.

15. A method for blood chemistry analysis comprising the steps of:
inserting a hypodermic syringe into a blood vessel of a patient;
withdrawing a sample into the syringe; and
sensing at least one blood component of said blood contained within said syringe.

16. The method of claim 15, further including the step of ejecting said blood back into the vessel of the patient from the syringe.

Amended claims is accordance with Rule 86(2) EPC.

1. A blood chemistry analysis device for use with a hypodermic syringe and a hypodermic needle, said hypodermic syringe having a hollow cylinder, a piston slidably mounted in said cylinder in a substantially airtight manner to form an enclosed chamber with a volume controlled by the position of said piston in said cylinder, and a syringe connector formed on the opposite end of said cylinder in communication with said enclosed chamber, said syringe connection being of the type that is adapted to mate with a needle connector formed on the proximal end of said hypodermic needle so that said hypodermic needle may be detachably mounted on said hypodermic syringe with the lumen of said needle in communication with the enclosed chamber said hypodermic syringe, said blood chemistry analysis device comprising an adapter having a hollow cavity, a syringe connector of the type formed on the cylinder of said hypodermic syringe, said syringe connector being in communication with said hollow cavity, a needle connector of the type formed on the proximal end of said needle, said needle connector being in communication with said hollow cavity and spaced from the syringe connector of said adapter by said hollow cavity, and a blood chemistry sensor mounted in said hollow cavity whereby said adapter may be placed between said hypodermic needle on the syringe connector of said adapter and by mounting the needle connector of said adapter on sdaid hypodermic syringe so that blood drawn into said hypodermic syringe from said needle flows through the hollow cavity of said adapter and makes contact with said blood chemistry sensor to analyse a chemical parameter of said blood.

2. The blood chemistry analysis device of claim 1 wherein said blood chemistry sensor comprises a plurality of separate sensors each of which senses a different blood chemical parameter.

3. The blood chemistry analysis device of claim 1 wherein said blood chemistry sensor comprises a single sensing device sensing a plurality of blood chemical parameters.

4. The blood chemistry analysis device of claim 1 wherein the hollow cavity of said adapter is formed by a hollow cylindrical body, and wherein said syringe connector and said needle connector are formed at opposite ends of said cylindrical body.

FIG.1

FIG.2

FIG.3

ELECTRONICS 138

ELECTRONICS 138

ELECTRONICS 338

FIG.4

344

404

402

408

406

FIG.5

502

506
512
510
500
520
514
508

504

522

142

144

140
130
132
134
136
137

ELECTRONICS
138

FIG.6

610

608
606

140  142  144

612
600

602

614

130
132
134

604

ELECTRONIC
READ OUT
138

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | US-A-4 553 552 (J.M. VALDDESPINO et al.) <br> * Abstract; column 2, lines 9-28,58-61; figures 1-5 * | 1-4,12, 15 | A 61 B 5/14 <br> G 01 N 33/48 |
| X | AT-A- 376 117 (O. PROHASKA) <br> * Page 2, lines 38-47; page 3, lines 29-42; page 5, claims 1,2,4,8; figure 2 * | 1-8,12, 13,15 | |
| X | DE-A-2 850 546 (HERAEUS-CHRIST GmbH) <br> * Pages 1-4; claims 1,4,9,16; page 5, lines 14-19; page 7, line 1 - page 8, line 14; page 12, lines 16-27; figures 1-7 * | 1-4,10- 13,15 | |
| A | WO-A-8 704 914 (AFFILIATED INNOVATION MANAGEMENT) <br> * Abstract; page 4, lines 17-22; page 5, line 26 - page 6, line 8; page 6, lines 23-33; page 14, lines 1-26; figures 1-3 * | 1,5,8, 10-12, 15,16 | TECHNICAL FIELDS SEARCHED (Int. Cl.4) |
| A | DE-A-2 622 959 (P. VAN BUREN Jr.) <br> * Page 5, line 33 - page 6, line 15; figure 3 * | 1,10-15 | A 61 B <br> G 01 N |

The present search report has been drawn up for all claims

| Place of search | Date of completion of the search | Examiner |
|---|---|---|
| THE HAGUE | 28-12-1988 | RIEB K.D. |